# FASCICULE DE BREVET EUROPEEN

(11) **EP 1 052 289 B1**
(45) Date de publication et mention de la délivrance du brevet: **14.07.2004**
(21) Numéro de dépôt: 00401239.9
(22) Date de dépôt: 05.05.2000
(51) Int. Cl.: C12P 7/18

(54) **Procédé de production d'arabitol par fermentation continue**
Verfahren zur Herstellung von Arabitol durch kontinuierliche Gärung
Process for the production of arabitol by continuous fermentation

(30) Priorité: 11.05.1999 FR 9905995
(43) Date de publication de la demande: 15.11.2000
(73) Titulaire: Roquette Frères, 62136 Lestrem (FR)
(72) Inventeur: Duflot, Pierrick, 62136 La Couture (FR); Lanos, Pierre, 59480 La Bassee (FR); Machu, Fabrice, 62400 Locon (FR); Segueilha, Laurent, 59130 Lambersart (FR)
(74) Mandataire: Touati, Catherine

(56) Documents cités:
- EP-A- 0 327 342
- US-A- 2 986 495
- ESCALANTE J ET AL.: "Production of arabitol from glucose by Hansenula polymorpha" JOURNAL OF FERMENTATION AND BIOENGINEERING, JP, SOCIETY OF FERMENTATION TECHNOLOGY, vol. 70, no. 4, 1990, page 228-231 XP000565376 ISSN: 0922-338X
- VAN ZYL P.J. ET AL: "Water relations of polyol accumulation by Zygosaccharomyces rouxii in continuous culture." APPLIED MICROBIOLOGY AND BIOTECHNOLOGY, vol. 33, no. 1, 1990, pages 12-17, XP000866383

## Description

La présente invention concerne un procédé de production d'arabitol par fermentation continue d'au moins un sucre par des micro-organismes producteurs d'arabitol.

Elle a plus particulièrement pour objet un procédé de production d'arabitol par fermentation continue d'au moins un sucre par des micro-organismes producteurs d'arabitol qui met en oeuvre au moins deux zones de fermentation reliées l'une à l'autre.

Plus précisément, le procédé conforme à l'invention consiste à réaliser la production d'arabitol dans une première zone de fermentation, en favorisant la consommation d'une partie du sucre introduit dans le milieu de fermentation qu'elle contient, puis à transférer une partie dudit milieu de fermentation dans une seconde zone de fermentation, tout en maintenant constant le volume de ladite première zone par ajout de sucre, et à poursuivre la production d'arabitol dans la seconde zone de fermentation de manière à consommer le sucre résiduel.

On procède ensuite, dans la seconde zone de fermentation, à la séparation en continu du milieu de fermentation à faible teneur en sucre résiduel ainsi obtenu en une fraction concentrée en micro-organismes et une autre fraction soluble enrichie en arabitol, et éventuellement au recyclage desdits micro-organismes à l'entrée de la première zone de fermentation.

La préparation industrielle de l'arabitol repose principalement sur des procédés fermentaires.

De manière générale, les procédés micro-biologiques de production d'arabitol peuvent mettre en oeuvre deux modes de fermentation différents : le mode discontinu et le mode continu.

Dans le mode discontinu, deux techniques sont classiquement utilisées: la technique de fermentation discontinue proprement dite (ou batch) et la technique de fermentation discontinue alimentée (ou fed batch).

Dans la technique de fermentation discontinue proprement dite, tous les substrats nécessaires à l'alimentation des micro-organismes sont introduits au début de la fermentation, et l'arabitol produit est extrait en fin de fermentation.

On entend par « substrats », l'ensemble des éléments nutritifs que l'on introduit dans le milieu de fermentation. Au sens de l'invention, les substrats sont principalement les sources carbonées (y compris le sucre) et azotées directement assimilables par les micro-organismes producteurs d'arabitol.

Cette technique de fermentation discontinue présente cependant l'inconvénient de demander de longs temps de fermentation, pour permettre la consommation totale du sucre introduit dans le milieu de fermentation, et de nécessiter la mise en oeuvre de grands volumes de milieu de fermentation, et donc de fermenteurs, pour obtenir des rendements de conversion satisfaisants. Ce qui se traduit par une productivité volumique médiocre.

De plus, il arrive que la variation de la concentration en l'un des constituants du milieu de fermentation affecte le rendement ou la productivité en arabitol.

Une solution consiste à mettre en oeuvre la seconde technique de fermentation discontinue, appelée fermentation discontinue alimentée.

Cette technique consiste notamment à introduire les substrats progressivement dans le milieu de fermentation.

L'avantage de cette technique est alors non seulement de pouvoir contrôler la concentration en substrats du milieu de fermentation, mais également de permettre la consommation totale du sucre introduit.

La technique de fermentation discontinue alimentée permet alors de résoudre les problèmes liés aux effets inhibiteurs potentiels de certains des constituants des substrats de la fermentation vis à vis de la croissance des micro-organismes ou de la production d'arabitol, ou de régler par exemple plus finement l'aération qui conditionne la production de l'arabitol.

Cependant, pour obtenir des rendement et productivité satisfaisants, il est nécessaire de mettre en oeuvre des concentrations élevées en substrats. Surtout, les durées de fermentation sont longues et il faut assurer un contrôle lourd et fastidieux des différentes étapes de la fermentation.

Ainsi, le procédé décrit par ESCALANTE et al. (in Journal of Fermentation and Bioengineering, 70-4, 228-231, 1990) pour la production d'arabitol par *Hansenula* en fermentation discontinue alimentée, ne permet pas d'atteindre des rendement et productivité industriellement intéressants.

En effet, même si l'arabitol est sélectivement produit, et si le procédé de fermentation discontinue alimentée permet d'assurer la consommation de tout le glucose introduit dans le milieu de fermentation, l'arabitol n'est obtenu qu'avec un rendement de l'ordre de 14 %.

Le second mode de fermentation classiquement mis en oeuvre pour la production d'arabitol par voie biologique, est le mode continu, comme par exemple décrit par JAKOBUS van ZYL et PRIOR dans Appl. Microbiol. Biotechnol. (1990), 33, 12-17.

Dans le mode continu, tous les substrats de fermentation sont ajoutés de manière continue dans le fermenteur, et des fractions du milieu de fermentation sont extraites avec le même débit que l'apport en substrats, de manière à travailler à volume constant.

Ce mode de fermentation permet un gain appréciable de rendement et de productivité, mais ne permet pas d'obtenir un produit de haute pureté, puisque l'arabitol soutiré est obligatoirement contaminé par les substrats réintroduits en cours de fermentation.

Par conséquent, l'inconvénient majeur des procédés de fermentation en mode continu est la nécessité de purifier l'arabitol. En outre, les techniques de purification ne permettent que difficilement de séparer l'arabitol du sucre résiduel non assimilé par les micro-organismes.

De ce fait, on trouve classiquement associé à ces procédés de fermentation continus, des installations de purification complexes, lourdes et coûteuses.

Tous les efforts des spécialistes en fermentation portent donc sur la recherche des conditions opératoires qui conduisent à concilier les meilleurs rendement et productivité en arabitol, avec une faible teneur en sucre résiduel qui autorise une purification peu coûteuse et aisée de l'arabitol produit.

La présente invention a donc pour but de résoudre ce problème de production d'arabitol de pureté satisfaisante avec d'excellents rendement et productivité par la mise en oeuvre d'une technique de fermentation en mode continu particulière.

Le procédé de production d'arabitol mis au point par la société Demanderesse consiste à réaliser la fermentation d'au moins un sucre par les micro-organismes producteurs d'arabitol dans deux zones de fermentation.

L'invention porte plus particulièrement sur un procédé de production d'arabitol par fermentation continue d'au moins un sucre par des micro-organismes producteurs d'arabitol, caractérisé par le fait que l'on :
a) conduit la production d'arabitol dans une première zone de fermentation, comprenant au moins un fermenteur, de manière à ce qu'une partie du sucre introduit dans le milieu de fermentation soit consommée par lesdits micro-organismes,
b) transfère une partie du milieu de fermentation ainsi obtenu dans une seconde zone de fermentation, comprenant au moins un fermenteur, en maintenant le volume constant dans la première zone de fermentation par ajout de sucre,
c) poursuit la production d'arabitol dans ladite seconde zone de fermentation de manière à consommer le sucre résiduel du milieu de fermentation,
d) sépare en continu le milieu de fermentation de ladite seconde zone de fermentation ainsi obtenu en une fraction concentrée en micro-organismes et une autre fraction soluble enrichie en arabitol.
e) collecte l'arabitol ainsi produit.

La première étape du procédé conforme à l'invention consiste à conduire la production d'arabitol dans une première zone de fermentation, comprenant au moins un fermenteur, de manière à ce qu'une partie du sucre introduit dans le milieu de fermentation, soit consommée par lesdits micro-organismes.

On choisit avantageusement les micro-organismes parmi les levures osmotolérantes, naturelles ou modifiées, productrices d'arabitol à partir de sucre comme source de carbone directement assimilable.

Par « levures osmotolérantes », on entend des levures capables de supporter des pressions osmotiques importantes et appartenant aux genres *Yamadazyma, Debaromyces, Hansenula, Candida, Zygosaccharomyces* et *Saccharomyces*... sans que cette liste ne soit limitative.

Par « levures osmotolérantes naturelles », on entend des levures osmotolérantes qui sont isolées de leur environnement pour leurs capacités naturelles à produire un métabolite donné, ici l'arabitol.

Par « levures osmotolérantes modifiées », on entend des levures osmotolérantes dont les capacités naturelles à produire un métabolite donné, ici l'arabitol, ont été optimisées par la mise en oeuvre de techniques de mutagenèse (aléatoires ou dirigées) ou de techniques de biologie moléculaire.

Par « sucre », on entend dans la présente invention toutes les sources carbonées directement assimilables par les micro-organismes producteurs d'arabitol.

Un tel sucre est par exemple choisi dans le groupe constitué par le glucose, le galactose, le saccharose, l'arabinose, le glycérol, le fructose, le maltose, le xylulose, le ribulose, le mannitol, le myo-inositol, l'éthanol, l'amidon et le maltose, seuls ou en mélange entre eux.

La première zone de fermentation est mise en oeuvre de façon à assurer une production élevée en arabitol, en favorisant la consommation d'une partie du sucre introduit dans le milieu de fermentation.

On entend par « une partie du sucre » au sens de l'invention, une quantité de sucre d'au moins 50 % en poids, de préférence d'au moins 80 % en poids, et de manière plus préférentielle encore, d'au moins 90 % en poids.

Dans un premier mode préférentiel de réalisation du procédé conforme à l'invention, la production d'arabitol est initiée dans la première zone de fermentation en mode discontinu, tous les substrats de fermentation, y compris le sucre, et les micro-organismes étant introduits simultanément.

Dans un second mode préférentiel de réalisation du procédé conforme à l'invention, la production d'arabitol est initiée dans la première zone de fermentation en mode discontinu alimenté, l'alimentation en sucre des micro-organismes producteurs d'arabitol étant conduite de manière progressive.

Un mode d'alimentation discontinu alimenté est avantageusement choisi pour démarrer la fermentation dans le procédé conforme à l'invention.

Ce mode d'alimentation discontinu permet d'assurer le remplissage progressif de la première zone de fermentation en substrats de manière à maintenir un taux élevé de croissance des micro-organismes, une bonne conversion du sucre en arabitol et une concentration en sucre du milieu de fermentation à une valeur tolérée par le micro-organisme producteur d'arabitol considéré.

On entend par « concentration en sucre tolérée par le micro-organisme », une concentration en sucre minimale qui n'inhibe ni la croissance du dit micro-organisme, ni sa production d'arabitol.

De manière générale, pour tous les micro-organismes producteurs d'arabitol, les conditions mises en oeuvre sont réglées de manière à éviter une consommation totale du sucre.

La production élevée d'arabitol dans la première zone de fermentation conduit généralement au maintien de quantités également relativement élevées en sucre résiduel non consommé, dont l'importance est fonction des micro-organismes considérés. De manière générale, ce taux de sucre résiduel est au moins égal à 2,5 % en poids.

La deuxième étape du procédé conforme à l'invention consiste alors à transférer une partie du milieu de fermentation ainsi obtenu dans une seconde zone de fermentation, comprenant au moins un fermenteur, tout en maintenant le volume constant dans la première zone de fermentation par ajout de sucre.

On effectue ainsi le transfert d'une partie du milieu de fermentation de la première zone de fermentation à la seconde, lorsque ledit milieu de fermentation est enrichi en arabitol, mais contient cependant encore du sucre résiduel non consommé, en quantité telle qu'elle rend la purification de l'arabitol produit lourde et complexe.

Ce transfert s'accompagne d'une alimentation continue en sucre dans la première zone de fermentation, ceci afin non seulement de maintenir le volume constant, mais surtout de continuer la production en arabitol avec une productivité et un rendement de conversion au moins égaux aux valeurs obtenues lors de la phase d'initiation de la production d'arabitol décrite ci-dessus.

On choisit avantageusement d'alimenter en continu la première zone de fermentation avec les substrats, plutôt qu'uniquement avec du sucre, comme exemplifié ci-après.

La troisième étape du procédé conforme à l'invention consiste à poursuivre la production d'arabitol dans la seconde zone de fermentation de manière à consommer le sucre résiduel de la partie du milieu de fermentation en provenance de la première zone de fermentation.

La seconde zone de fermentation est donc mise en oeuvre à la fois pour poursuivre la production d'arabitol, mais surtout pour terminer la consommation du sucre qui contamine la partie du milieu de fermentation transférée de la première zone de fermentation à la seconde, de manière à ne laisser qu'un faible taux de sucre résiduel.

Une faible teneur en sucre résiduel s'entend avantageusement d'une concentration en sucre dans le milieu de fermentation au plus égale à 2 % en poids, et de préférence au plus égale à 1 % en poids.

Dans un mode préférentiel du procédé conforme à l'invention, on conduit alors cette étape en mettant en oeuvre des fermenteurs de taille différente, les fermenteurs de la seconde zone de fermentation présentant un volume inférieur à ceux de la première zone de fermentation.

Dans un mode encore plus préférentiel du procédé conforme à l'invention, on choisit de n'utiliser qu'un fermenteur par zone de fermentation.

La quatrième étape du procédé conforme à l'invention consiste à séparer en continu le milieu de fermentation à faible teneur en sucre résiduel ainsi obtenu en une fraction concentrée en micro-organismes et une autre fraction soluble enrichie en arabitol.

On réalise la séparation continue entre les micro-organismes et la fraction enrichie en arabitol par tout moyen connu par ailleurs par l'homme du métier, par exemple par micro-filtration, en utilisant des membranes dont le diamètre des pores est adapté à la taille du micro-organisme considéré ou par centrifugation dans un intervalle de 1.000 à 10.000 G, et de manière préférentielle par micro-filtration, comme exemplifié ci-après.

La solution clarifiée, enrichie en arabitol, obtenue au terme de cette étape de séparation, constitue l'arabitol produit.

Il a été également établi par la société Demanderesse qu'il peut être avantageux de recycler la fraction enrichie en micro-organismes à l'entrée de la première zone de fermentation.

L'avantage de cette étape de recyclage des micro-organismes est de réintroduire une biomasse dont les capacités de fermentation sont entières, et d'éviter par là même un retard de mise en oeuvre qu'occasionnerait la préparation d'une nouvelle culture de micro-organismes producteurs d'arabitol.

La récupération de l'arabitol produit est effectuée par exemple par concentration à une valeur de l'ordre de 20 % et plus, et peut être suivi par une étape de cristallisation par toute méthode connue en soi par l'homme du métier.

D'autres caractéristiques et avantages de l'invention apparaîtront à la lecture des exemples non limitatifs décrits ci-dessous.

### Exemple 1

Les deux zones de fermentation mises en oeuvre comportent chacune un fermenteur, mais de taille différente. Le premier fermenteur de marque CHEMAP® présente un volume de 20 l (17 l utiles), et le second de même marque, un volume de 5 l (4 l utiles).

Une préculture du micro-organisme *Yamadazyma ohmeri*, souche ATCC 20209, est tout d'abord réalisée dans un autre fermenteur de 20 l.

Le milieu de préculture est constitué de glucose à 50 g/l, de liqueur de corn steep à 8 g/l, de KH₂PO₄ à 2 g/l et de MgSO₄ à 1 g/l. La préculture est réalisée à 30°C, sous une agitation à 600 rpm, une aération de 1 vvm, pendant 12 h, et une régulation de pH à 4,5 avec du NH₄OH à 20%.

Le fermenteur de la première zone de fermentation est ensemencée avec 1 l de cette préculture. On complète à 6 l avec un milieu renfermant du glucose à 50 g/l, de la liqueur de corn steep à 8 g/l, du KH₂PO₄ à 2,5 g/l, du MgSO₄ à 1 g/l et du FeSO₄ à 0,05 g/l.

La température est fixée à 30°C. Le pH est régulé à 4,5 jusque 35 h à l'aide de NH₄OH 20 %, puis la régulation est fixée à 3 à l'aide de KOH 5 N.

L'ajout de glucose est assuré comme indiqué dans le tableau I suivant. La biomasse est estimée par la mesure de l'absorbance (densité optique ou DO) du milieu à 620 nm.

Pour *Yamadazyma ohmeri*, une unité d'absorbance à 600 nm équivaut à une biomasse de l'ordre de 0,2 g/l.

**Tableau I**

| Temps (h) | DO | Glucose (g/l) | Arabitol (g/l) | Volume (l) | Ajouts de glucose |
|---|---|---|---|---|---|
| 0 | 14 | 50 | 0 | 6 | |
| 10 | 49 | 29 | 8 | 6 | 500 g dans 5 l |
| 20 | 91 | 30 | 15 | 10 | 1200 g dans 5 l |
| 30 | 154 | 30 | 31 | 15 | 1300 g dans 3 l |
| 40 | 190 | 29 | 60 | 17 | |

Après 40 h de fermentation, le volume utile du fermenteur de la première zone de fermentation est atteint, et la concentration en glucose résiduel est de 29 g/l.

4 l de milieu de fermentation de ce premier fermenteur sont alors transférés dans le fermenteur de la seconde zone de fermentation, et 800 g de glucose sont alors ajoutés dans un volume de 2 l dans le premier fermenteur. La température de la seconde zone de fermentation est fixée à 38°C, et le pH est régulé à 3,5 à l'aide de KOH 5 N.

Les fermenteurs évoluent ensuite séparément jusque 46 h, heure à partir de laquelle on effectue le premier prélèvement d'arabitol dans le second fermenteur.

A partir de 46 h, le fermenteur de la première zone de fermentation est alimenté en glucose à 250 g/l à un débit de l'ordre de 0,4 l/h, et en liqueur de corn steep à 50 g/l à un débit de l'ordre de 10 ml/h.

Le milieu circule de la première zone de fermentation à la seconde à l'aide d'une pompe PCM®, puis de la seconde zone de fermentation à un module de filtration tangentielle MEMBRALOX®, qui permet de récupérer un perméat riche en arabitol et un rétentat riche en micro-organismes.

Le rétentat est alors réintroduit à l'entrée de la première zone de fermentation.

De façon à maintenir les niveaux dans les deux fermenteurs, le débit du perméat sortant est égal au débit d'alimentation en glucose.

Ce débit évolue au cours du temps en fonction de la vitesse de consommation du glucose par les micro-organismes dans les fermenteurs. A 200 h, l'alimentation est stoppée, et le fermenteur de la deuxième zone de fermentation est vidé.

On laisse se terminer la fermentation dans le fermenteur de la première zone de fermentation pendant 10 h supplémentaires, avant de traiter le milieu sur le module de filtration tangentielle.

Le tableau II suivant présente les mesures effectuées après transfert du milieu de fermentation de la première zone de fermentation à la seconde.

**Tableau II**

| | Premier fermenteur | | | | Second fermenteur | | | |
|---|---|---|---|---|---|---|---|---|
| Temps (h) | DO | Glucose (g/l) | Arabitol (g/l) | Volume (l) | DO | Glucose (g/l) | Arabitol (g/l) | Volume (l) |
| 40 | 190 | 29 | 60 | 17 | | | | |
| 40,1 | 165 | 75 | 57 | 15 | 190 | 28 | 61 | 4 |
| 46 | 170 | 39 | 74 | 15 | 190 | 10 | 74 | 4 |
| 60 | 173 | 25 | 95 | 15 | 175 | 5 | 102 | 4 |
| 80 | 177 | 26 | 107 | 15 | 174 | 6 | 115 | 4 |
| 100 | 182 | 25 | 111 | 15 | 183 | 4 | 119 | 4 |
| 120 | 184 | 25 | 113 | 15 | 186 | 5 | 123 | 4 |
| 140 | 191 | 27 | 112 | 15 | 190 | 6 | 121 | 4 |
| 160 | 193 | 25 | 108 | 15 | 194 | 6 | 119 | 4 |
| 180 | 194 | 28 | 105 | 15 | 189 | 7 | 114 | 4 |
| 200 | 201 | 25 | 102 | 15 | 194 | 9 | 110 | 4 |
| 210 | 198 | 1 | 115 | 15 | | | | |

Globalement, 8,8 kg d'arabitol sont récupérés pour 18,7 kg de glucose mis en oeuvre, soit un rendement de 47 % et une productivité de 2,2 g/l/h.

En fermentation discontinue réalisée dans les mêmes conditions de milieu de production, avec le même apport en glucose, le rendement est de 40 % et la productivité n'est que de 1,5 g/l/h.

Le procédé conforme à l'invention permet ainsi d'assurer de bien meilleurs rendement et productivité.

En outre, le faible taux de glucose résiduel permet de récupérer un arabitol présentant une pureté tout à fait satisfaisante.

### Exemple 2

On conduit la préparation d'arabitol dans des conditions de fermentation identique à celles de l'exemple 1, mais en utilisant un micro-organisme *Yamadazyma ohmeri*, souche ATCC 20209 modifié par la technique de mutagenèse aléatoire classique aux ultraviolets (en mettant en oeuvre au moins un cycle de mutagenèse aux ultraviolets avec enrichissement à la nystatine), la souche étant sélectionnée pour son aptitude à produire l'arabitol avec un rendement de 8 à 10 points supérieur à la souche mère.

Après l'obtention d'une préculture de ladite souche modifiée, et transfert dans la première zone de fermentation comme indiqué dans l'exemple 1, l'ajout de glucose est assuré comme présenté dans le tableau III suivant.

**Tableau III**

| Temps (h) | DO | Glucose (g/l) | Arabitol (g/l) | Volume (l) | Ajouts de glucose |
|---|---|---|---|---|---|
| 0 | 15 | 50 | 0 | 6 | |
| 10 | 50 | 19 | 8 | 6 | 500 g dans 5 l |
| 20 | 102 | 31 | 15 | 10 | 1200 g dans 5 l |
| 30 | 164 | 29 | 36 | 15 | 1300 g dans 3 l |
| 40 | 204 | 28 | 70 | 17 | |

Après 40 h de fermentation, le volume utile du fermenteur de la première zone de fermentation est atteint, et la concentration en glucose résiduel est de 28 g/l.

4 1 de milieu de fermentation de ce premier fermenteur sont alors transférés dans le fermenteur de la seconde zone de fermentation, et 800 g de glucose sont alors ajoutés dans un volume de 2 l dans le premier fermenteur.

La température de la seconde zone de fermentation est fixée à 38°C, et le pH est régulé à 3,5 à l'aide de KOH 5N.

Les fermenteurs évoluent ensuite séparément jusque 46 h, heure à partir de laquelle on effectue le premier prélèvement d'arabitol dans le second fermenteur.

A partir de 46 h, le fermenteur de la première zone de fermentation est alimenté en glucose à 250 g/l à un débit de l'ordre de 0,4 l/h, et en liqueur de corn steep à 50 g/l à un débit de l'ordre de 10 ml/h.

Le tableau IV suivant présente les mesures effectuées après transfert du milieu de fermentation de la première zone de fermentation à la seconde.

**Tableau IV**

| | Premier fermenteur | | | | Second fermenteur | | | |
|---|---|---|---|---|---|---|---|---|
| Temps (h) | DO | Glucose (g/l) | Arabitol (g/l) | Volume (l) | DO | Glucose (g/l) | Arabitol (g/l) | Volume (l) |
| 40 | 204 | 28 | 70 | 17 | | | | |
| 40,1 | 180 | 76 | 62 | 15 | 204 | 28 | 70 | 4 |
| 46 | 191 | 39 | 85 | 15 | 196 | 12 | 90 | 4 |
| 60 | 204 | 23 | 121 | 15 | 200 | 5 | 128 | 4 |
| 80 | 212 | 22 | 141 | 15 | 210 | 7 | 152 | 4 |
| 100 | 222 | 24 | 150 | 15 | 225 | 6 | 163 | 4 |
| 120 | 225 | 26 | 151 | 15 | 224 | 5 | 165 | 4 |
| 140 | 230 | 25 | 150 | 15 | 230 | 6 | 166 | 4 |
| 160 | 227 | 24 | 149 | 15 | 225 | 6 | 164 | 4 |
| 180 | 228 | 21 | 148 | 15 | 227 | 8 | 163 | 4 |
| 200 | 231 | 25 | 145 | 15 | 235 | 7 | 161 | 4 |
| 210 | 230 | 1 | 164 | 15 | | | | |

Globalement, 12 kg d'arabitol sont récupérés pour 19 kg de glucose mis en oeuvre, soit un rendement de 63 % et une productivité de 4 g/l/h.

En fermentation discontinue réalisée dans les mêmes conditions de milieu de production, avec le même apport en glucose, le rendement est de 55 % et la productivité n'est que de 2,5 g/l/h.

Le procédé conforme à l'invention permet ainsi d'assurer de bien meilleurs rendement et productivité.

En outre, le faible taux de glucose résiduel permet toujours de récupérer un arabitol présentant une pureté tout à fait satisfaisante.

### Exemple 3

Les deux zones de fermentation sont mises en oeuvre comme dans l'exemple 1.

Une préculture du micro-organisme *Candida polymorpha*, souche ATCC 20213 naturelle, est tout d'abord réalisée dans un fermenteur CHEMAP® de 20 l, dans les mêmes conditions que pour la souche de *Y. ohmeri* de l'exemple 1.

Le fermenteur de la première zone de fermentation est ensemencée avec 1 l de cette préculture.

On complète à 6 l avec un milieu renfermant du glucose à 50 g/l, de l'extrait de levure à 3 g/l, du KH₂PO₄ à 2 g/l et du MgSO₄ à 1 g/l.

La température est fixée à 30°C. Le pH est régulé à 4,5 jusque 30 h à l'aide de NH₄OH 20%, puis à l'aide de KOH 5 N.

L'ajout de glucose est contrôlé comme indiqué dans le tableau III suivant.

La biomasse est estimée par la mesure de l'absorbance (densité optique) du milieu à 620 nm.

Pour *Candida polymorpha*, une unité d'absorbance à 600 nm équivaut à une biomasse de l'ordre de 0,22 g/l.

**Tableau V**

| Temps (h) | DO | Glucose (g/l) | Arabitol (g/l) | Volume (l) | Ajouts de glucose |
|---|---|---|---|---|---|
| 0 | 10 | 50 | 0 | 6 | |
| 10 | 45 | 21 | 3 | 6 | 500 g dans 5 l |
| 20 | 85 | 27 | 8 | 10 | 1200 g dans 5 l |
| 30 | 144 | 33 | 19 | 15 | 1300 g dans 3 l |
| 40 | 174 | 30 | 30 | 17 | |

A 40 h de fermentation, le volume utile du fermenteur de la première zone de fermentation est atteint, et la concentration en glucose résiduel est de 30 g/l.

4 l de milieu de fermentation de ce premier fermenteur sont alors transférés dans le fermenteur de la seconde zone de fermentation, et 800 g de glucose sont ajoutés dans un volume de 2 l dans le premier fermenteur.

La température de la seconde zone de fermentation est fixée à 37 °C, et le pH est régulé à 4,5 par KOH 5 N.

Les fermenteurs évoluent ensuite séparément jusque 46 h, heure à partir de laquelle on effectue le premier prélèvement d'arabitol dans le second fermenteur.

A partir de 46 h, le fermenteur de la première zone de fermentation est alimenté en glucose à 250 g/l à un débit de l'ordre de 0,4 l/h, et en liqueur de corn steep à 50 g/l à un débit de l'ordre de 10 ml/h.

Le tableau IV suivant présente les mesures effectuées après transfert du milieu de fermentation de la première zone de fermentation à la seconde.

**Tableau VI**

| | Premier fermenteur | | | | Second fermenteur | | | |
|---|---|---|---|---|---|---|---|---|
| Temps (h) | DO | Glucose (g/l) | Arabitol (g/l) | Volume (l) | DO | Glucose (g/l) | Arabitol (g/l) | Volume (l) |
| 40 | 174 | 30 | 30 | 17 | | | | |
| 40,1 | 150 | 77 | 27 | 15 | 174 | 29 | 30 | 4 |
| 46 | 160 | 44 | 34 | 15 | 174 | 14 | 36 | 4 |
| 60 | 165 | 31 | 46 | 15 | 164 | 11 | 50 | 4 |
| 80 | 167 | 29 | 51 | 15 | 169 | 8 | 56 | 4 |
| 100 | 172 | 23 | 52 | 15 | 171 | 6 | 58 | 4 |
| 120 | 176 | 27 | 51 | 15 | 174 | 5 | 59 | 4 |
| 140 | 180 | 29 | 50 | 15 | 179 | 7 | 57 | 4 |
| 160 | 184 | 28 | 49 | 15 | 181 | 7 | 55 | 4 |
| 180 | 183 | 24 | 50 | 15 | 177 | 8 | 55 | 4 |
| 200 | 186 | 26 | 47 | 15 | 182 | 8 | 53 | 4 |
| 210 | 185 | 1 | 54 | 15 | | | | |

Globalement, 4,1 kg d'arabitol sont récupérés pour 17,4 kg de glucose mis en oeuvre, soit un rendement de 23,6 % et une productivité de 1 g/l/h.

En fermentation discontinue réalisée dans les mêmes conditions de milieu de production, avec le même apport en glucose, le rendement est de 16,5 % et la productivité n'est que de 0,6 g/l/h.

Le procédé conforme à l'invention permet ainsi, pour une souche considérée, d'assurer de bien meilleurs rendement et productivité que les procédés classiquement mis en oeuvre.

En outre, le faible taux de glucose résiduel permet de récupérer un arabitol présentant ici encore une pureté tout à fait satisfaisante.

## Revendications

1. Procédé de production d'arabitol par fermentation continue d'au moins un sucre par des micro-organismes producteurs d'arabitol, **caractérisé par le fait que** l'on :
a) conduit la production d'arabitol dans une première zone de fermentation, comprenant au moins un fermenteur, de manière à ce qu'une partie du sucre introduit dans le milieu de fermentation soit consommée par lesdits micro-organismes,
b) transfère une partie du milieu de fermentation ainsi obtenu dans une seconde zone de fermentation, comprenant au moins un fermenteur, tout en maintenant le volume constant dans la première zone de fermentation par ajout de sucre,
c) poursuit la production d'arabitol dans ladite seconde zone de fermentation de manière à consommer le sucre résiduel du milieu de fermentation,
d) sépare en continu le milieu de fermentation de ladite seconde zone de fermentation ainsi obtenu en une fraction concentrée en micro-organismes et une autre fraction soluble enrichie en arabitol,
e) collecte l'arabitol ainsi obtenu.

2. Procédé selon la revendication 1, **caractérisé par le fait que** l'on recycle à l'entrée de la première zone de fermentation ladite fraction concentrée en micro-organismes.

3. Procédé selon l'une ou l'autre des revendications 1 et 2 **caractérisé par le fait que** la partie du sucre consommé par les micro-organismes producteurs d'arabitol dans la première zone de fermentation est d'au moins 50 % en poids, de préférence d'au moins 80 % en poids, et plus préférentiellement encore d'au moins 90 % en poids du sucre introduit dans le milieu de fermentation.

4. Procédé selon l'une ou l'autre des revendications 1 et 3, **caractérisé par le fait que** la teneur en sucre résiduel dans la seconde zone de fermentation est d'au plus 2 % en poids, de préférence d'au plus 1 % en poids.

5. Procédé selon l'une quelconque des revendications 1 à 4, **caractérisé par le fait que** les fermenteurs de la seconde zone de fermentation présentent un volume inférieur à ceux des fermenteurs de la première zone.

6. Procédé selon la revendication 5, **caractérisé par le fait que** la première et la seconde zone de fermentation comprennent chacune un seul fermenteur.

7. Procédé selon l'une quelconque des revendications 1 à 6, **caractérisé par le fait que** les micro-organismes producteurs d'arabitol sont choisis dans le groupe constitué des levures osmotolérantes naturelles ou modifiées du genre *Yamadazyma, Debaromyces, Hansenula, Candida, Zygosaccharomyces* et *Saccharomyces*.

8. Procédé selon l'une quelconque des revendications 1 à 7, **caractérisé par le fait que** le sucre est choisi parmi les sources carbonées directement assimilables par les micro-organismes producteurs d'arabitol, et est préférentiellement le glucose.

## Claims

1. A method of producing arabitol by continuous fermentation of at least one sugar by arabitol-producing micro-organisms **characterized in that**:
a) arabitol is produced in a fist fermentation area comprising at least one fermenter, so that part of the sugar introduced into the fermentation medium is consumed by said micro-organisms,
b) part of the fermentation medium thus obtained is transferred into a second fermentation area comprising at least one fermenter, the volume being maintained constant in the first fermentation area by adding sugar,
c) production of arabitol is continued in said second fermentation area so as to consume the residual sugar of the fermentation medium,
d) the fermentation medium of said second fermentation area thus obtained is separated continuously into a fraction concentrated in micro-organisms and another soluble fraction enriched in arabitol, and
e) the arabitol thus obtained is collected.

2. A method according to claim 1 **characterized in that** said fraction concentrated in micro-organisms is recycled to the entry of the first fermentation area.

3. A method according to any one of claims 1 and 2 **characterized in that** the part of sugar consumed by the arabitol-producing micro-organisms in the first fermentation area is at least 50 wt.%, preferably at least 80 wt.% and still more preferably at least 90 wt.% of the sugar introduced into the fermentation medium.

4. A method according to any one of claims 1 and 3, **characterized in that** the content in residual sugar in the second fermentation area is at most 2 wt.% and preferably at most 1 wt.%.

5. A method according to any one of claims 1 to 4 **characterized in that** the fermenters of the second fermentation area have a smaller volume than the fermenters of the first area.

6. A method according to claim 5 **characterized in that** the first and second fermentation areas each comprise a single fermenter.

7. A method according to any one of claims 1 to 6 **characterized in that** the arabitol-producing micro-organisms are selected from the group constituted by natural or modified osmosis-tolerant yeasts of the genus *Yamadazyma, Debaromyces, Hansenula, Candida, Zygosaccharomyces* and *Saccharomyces*.

8. A method according to any one of claims 1 to 7 **characterized in that** the sugar is chosen from carbonaceous sources which can be directly assimilated by the arabitol-producing micro-organisms and is preferably glucose.

## Patentansprüche

1. Verfahren zur Herstellung von Arabit durch fortlaufendes Fermentieren von wenigstens einem Zucker durch Arabit herstellende Mikroorganismen, **gekennzeichnet durch** die Schritte, dass man:
a) die Produktion von Arabit in einer ersten Fermentationszone umfassend wenigstens einen Fermenter auf die Weise führt, dass ein Teil des Zuckers, der in das Fermentationsmedium eingebracht wird, **durch** die Mikroorganismen verbraucht wird,
b) einen auf diese Weise erhaltenen Teil des Fermentationsmediums in eine zweite Fermentationszone umfassend wenigstens einen Fermenter überträgt, ganz unter Konstanthalten des Volumens in der ersten Fermentationszone **durch** Hinzufügen von Zucker,
c) die Produktion von Arabit in der zweiten Fermentationszone auf die Weise fortsetzt, dass der Zucker verbraucht wird, der im Fermentationsmedium zurückgeblieben ist,
d) das auf diese Weise erhaltene Fermentationsmedium der zweiten Fermentationszone kontinuierlich in eine Fraktion trennt, in der Mikroorganismen konzentriert sind, und in eine andere lösliche Fraktion, die mit Arabit angereichert ist.
e) das auf diese Weise erhaltene Arabit sammelt.

2. Verfahren gemäß Anspruch 1, **gekennzeichnet durch** den Schritt, dass man die Fraktion, in der Mikroorganismen konzentriert sind, an den Eingang der ersten Fermentationszone rückführt.

3. Verfahren gemäß einem der Ansprüche 1 und 2, **gekennzeichnet durch** den Schritt, dass der Teil des Zuckers, der **durch** die Arabit herstellenden Mikroorganismen in der ersten Fermentationszone verbraucht wird, wenigstens 50 Gew.-%, vorzugsweise wenigstens 80 Gew.-%, stärker bevorzugt außerdem wenigstens 90 Gew.-% des Zuckers beträgt, der in das Fermentationsmedium eingebracht wird.

4. Verfahren gemäß einem der Ansprüche 1 und 3, **gekennzeichnet durch** den Schritt, dass der in der zweiten Fermentationszone zurückbleibende Zuckergehalt höchstens 2 Gew.-%, vorzugsweise höchstens 1 Gew.-% beträgt.

5. Verfahren gemäß einem beliebigen der Ansprüche 1 bis 4, **gekennzeichnet durch** den Schritt, dass die Fermenter der zweiten Fermentationszone ein kleineres Volumen als das der Fermenter der ersten Zone aufweisen.

6. Verfahren gemäß Anspruch 5, **gekennzeichnet durch** den Schritt, dass die erste und die zweite Fermentationszone jeweils einen einzigen Fermenter umfassen.

7. Verfahren gemäß einem beliebigen der Ansprüche 1 bis 6, **gekennzeichnet durch** den Schritt, dass die Arabit herstellenden Mikroorganismen aus der Gruppe bestehend aus natürlichen oder modifizierten osmotoleranten Hefen der Gattung *Yamadazyma, Debaromyces, Hansenula, Candida, Zygosaccharomyces* und *Saccharomyces* ausgewählt werden.

8. Verfahren gemäß einem beliebigen der Ansprüche 1 bis 7, **gekennzeichnet durch** den Schritt, dass der Zucker aus von Arabit herstellenden Mikroorganismen direkt assimilierbaren Kohlenstoffquellen ausgewählt wird und vorzugsweise Glukose ist.
